# EUROPEAN PATENT APPLICATION

(11) **EP 1 104 906 A1**
(43) Date of publication of application: **06.06.2001**
(21) Application number: 99121794.4
(22) Date of filing: 03.11.1999
(51) Int. Cl.: G06F 19/00

(54) **Process and apparatus for determining biomolecule interactions**

(71) Applicant: LION Bioscience AG, 69120 Heidelberg (DE)
(72) Inventor: Valencia, Alfonso, Dr., 28230 Madrid (ES); Cabaleiro, Florencio Pazos, 28029 Madrid (ES)
(74) Representative: Goddar, Heinz J., Dr.

(57) **Abstract**

Process for the determination of interacting biomolecules,
wherein
a) a first group is provided comprising sequences representing homologous biomolecules,
b) at least one second group is provided comprising sequences representing homologous biomolecules,
c) group correlation values between the sequences of the first group and the sequences of at least one second group are determined, and
d) the probability of the interaction of the sequence represented biomolecules is determined on the basis of the group correlation values.

## Description

The present invention relates to a process and a method for the determination of interacting biomolecules, use of such method, pairs of interacting biomolecules, data structure, computer readable medium, computer program, data base, computer system and device for simulating the interaction of biomolecules.

### Background of the invention

Recently the advances in molecular biology have led to a vast amount of information at the genetic level of many different organisms, including man. In fact, there are attempts under way to determine the entire chromosome sequences of various different organisms, such as the human DNA sequence. Some have even been completed, such as the DNA sequence of the eubacteria *Escherichia coli.* A larger number of organisms with smaller genomes such as viruses or eu- and archaebacterial organisms have been sequenced and their predicted or assigned gene sequences lie in various public or private databases.

Advances in technology have led to what many people refer to as "reverse genetics", the analysis of the DNA sequence and the subsequent prediction and analysis of protein function.

Often, upon sequence determination, functional predictions are based on homology analysis of a particular protein to other proteins with well defined biochemical characteristics and function. In such case, the function is well known and the interactions between this protein and further proteins have been characterized biochemically in detail. Undoubtedly, one of the major factors of being able to attribute function to a protein is the knowledge of the interactions and its binding *in vivo* and *in vitro.*

The knowledge of the interactions and binding capabilities of proteins is not only of importance for function prediction but even more so for industrially relevant applications such as lead target interactions.

It is possible to differentiate between two types of interactions, strong interactions typical of structural proteins forming functional complexes and weak interactions more related with the transient coupling of proteins performing a function or control a system.

Examples of the first type of interaction and complexes thus formed are the ribosome, the proteosome or the spliceosome. All of them are big molecular complexes, with fixed components as well as interchangeable components. Smaller complexes, like the dimer of tubulin that forms microtubuli, or the histone components of the nucleosome are examples of stable structural interactions responsible for important cellular functions.

Examples of weak interactions forming, e.g., transient complexes, are the interaction between Elongation Factor Tu, a key initiator of protein translation, and other proteins *(i.e.* EF-Ts) regulating the transition between different states of EF-Tu (GTP bound active state and GDP bound inactive) or the interaction between DnaK, a molecular chaperon responsible of protein folding and transport in bacterial cells, and DnaJ which is a co-chaperon responsible for the regulation of activation of DnaK. Such transient complexes are difficult to study experimentally since their physical interaction is weaker than in the more stable structural complexes and their life time as a complex can be very short.

The emergence of new techniques in biology, namely the "Yeast Two Hybrid-Analysis" herein referred to as Y2H-analysis, a molecular approach which is described in US Patent 5,283,173, Mass-spectrometry applied to 2D gels and DNA-chips (for e.g. expression arrays), is leading to the experimental establishment of relations between two or more proteins of a given genome. These techniques are often in a developmental stage, require careful experimental set-up and considerable investments. All are subject to different types of experimental errors, and the determination of protein complexes and interactions have a considerable margin of error. Thus the determination of interacting protein pairs from a given set of possible pairings has been very tedious and often unsuccessful.

Mass spectroscopy combined with 2D gel electrophoresis can be used for identifying such proteins that change expression level or degree of post translational modification during varying biological process, *e.g.* heat shock. In this case a very considerable financial investment must be made and the performance of gel electrophoresis is often times tedious and time consuming.

Gene expression may also be analyzed using DNA-arrays. Here, the expression of gene products is monitored in different states of a cell. As a result it is sometimes possible to predict interacting proteins. However such expression analyses requires a great investment into fairly novel technology.

Until recently it was thus only possible to determine protein interactions by applying "wet" laboratory techniques, as outlined above. A number of computational techniques have however recently appeared trying to tackle this problem.

In those cases in which the three-dimensional structure of the two interacting proteins is known, docking techniques can be applied for modeling of the possible protein complex. These techniques are limited in their success, require intensive computational resources and, more importantly, can only be applied when the structures have been determined previously experimentally.

Another problem is the prediction of interacting regions between two proteins without prior knowledge of their three-dimensional structures. Pazos et al., (1997) have addressed this. They show that it is possible to predict which amino acids of the polypeptide chain are close in space, based on the information in the sequence that has accumulated over evolutionary time and can be retrieved from multiple sequence alignments of similar genes or proteins *(e.g.* multiple sequence alignment of the protein family). Still, this approach is focused on the prediction of the region of interaction between pairs of proteins known to interact.

The computational efforts to identify which proteins are likely to interact in the absence of experimental information has only been addressed very recently by Dandeker et al. (Conservation of gene order: a fingerprint of proteins that physically interact, (1998) Dandekar, T., Snel, B., Huynen, M. and Bork, P. TIBS 23:324-328) identifying a relation between the proximity in the genome of the genes in bacterial genomes and the probability of physical interaction between their gene products. This approach is limited to a small set of genes whose proximity along the genome is conserved in many species.

Marcotte et al. (Marcotte et al., Science, vol. 285:751-753 (1999)) developed the idea of predicting protein-protein interaction for those multi-domain proteins that have different domain distributions in different genomes, *e.g.* a protein with two domains A and B from yeast may be represented in *E. coli* by two separate different proteins one containing domain A and the second containing domain B. The scope of this approach is limited to the few cases in which these types of molecular arrangements are met.

As outlined above there are "wet" laboratory techniques which enable the prediction of interacting sequences. These are often costly and time consuming. There are *in silico methods, i.e.* methods which can be adapted to run on computer devices for the prediction of interacting sites within a protein as well as interacting sites between two proteins known to interact but there is no method available for predicting the interaction biomolecules that takes only their sequences into account and that is generally applicable.

It was therefore an objective of the present invention to provide a process and/or a method which overcomes these limitations of the prior art.

It was also an objective of the present invention to provide for a process for identifying interacting biomolecules (i-2-hybrid process) that is reliable, cheap and avoids "wet" laboratory techniques.

It was therefore also an objective of the present invention to provide for a process that uses or requires only a primary sequence without the knowledge of a genome structure, the position of domains or other additional pieces of information.

It was a further objective of the present invention to provide for a process that may be applied to DNA, RNA and/or amino acid sequences.

It was further an objective of the present invention to provide for an apparatus for identifying interacting biomolecules.

It was further an objective of the present invention to create one or more databases containing information on such interacting sequence pairs by making use of the process of the present invention.

A further objective of the present invention is to provide a method for the determination of interacting biomolecules which comprises processing data. A still further object is to provide a data structure and a data base containing information and interacting sequence pairs.

Finally it is a further objective of the present application to provide a computer readable medium and a computer program product, respectively. Last but not least the objective of the present application is to provide a device for simulating the interaction of biomolecules represented by their sequences.

According to the invention this objective is solved by a process for the determination of interacting biomolecules and/or the simulation of the interaction of biomolecules wherein similar patterns of variation between two or more positions of at least two biomolecules are used.

According to the invention this objective is also solved by a process for the determination of interacting biomolecules which comprises the following steps:
a) a first group is provided comprising sequences representing homologous biomolecules,
b) at least one second group is provided comprising sequences representing homologous biomolecules,
c) group correlation values between the sequences of the first group and the sequences of at least one second group are determined, and
d) the probability of the interaction of the sequence represented biomolecules is determined on the basis of the group correlation values.

The objective is also solved by a method for the determination of interacting biomolecules which comprises processing data of at least a second set of data to output data
wherein each of the sets of data comprises independently and individually at least one or more elements,
wherein each of the elements represents the sequence of a biomolecule,
wherein the elements of a single set of data represent a group of homologous biomolecules
wherein the output data comprises at least one pair of elements with one part of the pair of elements comprising at least one element from the first set of data and the other part of the pair of elements comprising at least one element from the second set of data,
   whereby
   - a group correlation values data set is created comprising group correlation values which are determined between the sequences of the first set of data and at least the second set of data;
   - an interaction probability data set is created by retrieving group correlation values from the group correlation values data set and determining the probability of interaction of the biomolecules based on the group correlation values; and
   at least some of the elements from the first and at least the second set of data which have been used to create the group correlation values and the interaction probability therefrom form the output data.

Furthermore the objective is solved by the use of the inventive method for the simulation of biomolecule interaction.

The objective is furthermore solved by a data structure readable by a computer where said structure being generated by the inventive process or method.

The problem is also solved by a computer readable medium for embodying or storing therein data readable by a computer, said medium comprising one or more of the following:
a data structure generated by executing a process or a method;
Computer program code means which is adapted to cause a computer to execute the inventive process or method.

The problem is also solved by the database containing information on interesting sequence pairs generated by applying the inventive process or method.

The objective is also solved by a computer system comprising an execution environment for running their inventive process or inventive method.

The objective is furthermore solved by a device for simulating the interaction of biomolecules represented by the sequences which comprises
a loading device for making available the sets of data as specified in connection with the inventive method,
a processing device for performing the inventive method, and
an output device for receiving the output data generated by the processing device.

Finally the objective is also solved by pairs or complexes of interacting biomolecules determined in accordance with the inventive method or process.

In a preferred embodiment of the inventive process the probability of the interaction is calculated as predicted interaction value.

In a further preferred embodiment the interacting biomolecules are those with a positive predicted interaction value.

In a preferred embodiment of the inventive process any of the second group(s) is converted into the first group and the first group is converted into a second group and group correlation values between the sequences of this new first group and the sequences of any of the second group(s) which also comprises the former first group, are determined.

In a further embodiment of the inventive process site correlation values within each of the sequences within the first group and/or site correlation values within each of the sequences within the second group(s) are determined and said site correlation values are used for the calculation of the probability of interaction of the sequence represented biomolecules.

In a further more preferred embodiment of the inventive process the site correlation values are correlation values for substitutions within the sequences.

In a further embodiment of the inventive process each sequence of each of said groups is fused to each other to form fused sequences comprising at least one sequence of the first group and at least one sequence of any second group(s),
the correlation values within these fused sequences are determined, and
the correlation values are used as group correlation values for determining the probability of interaction.

In a further embodiment correlation values and preferably site correlation values are determined by
creating a position specific matrix containing the distances between pairs of sequences at that position whereby the distances are calculated by applying a standard distances matrix,
creating a combined matrix for two positions by calculating the covariation coefficient between corresponding entries of the position specific matrices, and
determining the correlation value for a pair of positions by averaging the correlation values of the combined matrix.

In a more preferred embodiment the standard distances matrix is the scoring matrix by McLachlan.

In an embodiment of the inventive method the probability of the interaction is calculated as predicted interaction value.

In a further embodiment the elements the predicted interaction value of which is positive, are interacting biomolecules.

In a still further embodiment any second set(s) of data is converted into the first set of data and the first set of data is converted into a second set of data, and
group correlation values are determined between the sequences of this new first set of data and the sequences of any of the second set(s).

In another embodiment of the inventive method site correlation values within each of the sequences within the first set of data and/or site correlation values within each of the sequences within the second set(s) of data are determined, and
said site correlation values form a set-specific site correlation value data set.

In a further embodiment of the inventive method the set-specific site correlation value data set is used to calculate the probability of interaction and/or to calculate the predicted interaction value of the sequence represented biomolecules.

In a further embodiment the site correlation values are correlation values for substitutions within the sequences.

In a still further embodiment a fused element set of data is generated by combining each element of the first set of data individually with each element of any of the second set(s) of data, and
attributing each fused element individually to the fused element set of data.

In a more preferred embodiment the correlation values are determined within the various positions of a single element of the fused element set of data, and
the correlation values are used as group correlation values for determining the probability of the interaction and/or predicted interaction value(s) of the biomolecules.

According to the inventive method the correlation values are determined in a preferred embodiment by
creating a position specific matrix containing the distances between pairs of sequences at that position whereby the distances are calculated by applying a standard distances matrix,
creating a combined matrix for two positions by calculating the covariation coefficient between corresponding entries of the position specific matrices or equivalent positions of the position specific matrices, and
determining the correlation value for a pair of positions by averaging the correlation values of the combined matrix.

More preferably the standard distances matrix is the scoring matrix by McLachlan.

In a further preferred embodiment of the inventive method the first set of data and/or the second set(s) of data are retrieved from a medium which is selected from the group comprising databanks, linked databanks, textual data and sets of data generated by an analytical instrument.

It is preferred that the set(s) of data comprise alligned sequences.

In another embodiment the output data are output control characters for a target medium.

In preferred embodiments of both the inventive method and process the sequences of the first group or second group(s) or first set of data or second set(s) of data are selected from the group comprising DNA sequences, RNA sequences and amino acid sequences.

In further preferred embodiments the number of sequences comprised in any of the groups or any of the sets of data is at least, preferably at least 11.

In another preferred embodiment of both the inventive method and process the sequences are homologous sequences.

In a more preferred embodiment the homologous sequences stem from different origins.

In an embodiment of both the inventive method and the inventive process the homologous sequences in the first set of data and in the second set of data stem from the same origin and/or the homologous sequence in the first group and in the second group stem from the same origin.

In further embodiments the homologous sequences are homologous genes.

In a more preferred embodiment the homologous genes are orthologs.

In a preferred embodiment of the inventive database the database is an organism/species specific database.

In an embodiment of use according to the present invention the interacting biomolecules are those with a positive predicted interaction value determined by a process or method according to the invention.

It has surprisingly been found that the yeast two hybrid system can be carried out in silico thus omitting the need for carrying out experiments in order to determine interacting biomolecules.

Herein homologues biomolecules represented by sequences means that the sequences of said biomolecules sequences are sequences that show sequence similarity. This sequence similarity may be high or low.

Herein, similar sequences are such sequences which are alignable when applying the CLUSTALW method (Higgins et al, see above) and such sequences that fall under the description of related sequences or derived sequences as found in
Doolittle, R. F. (1986). Of URFs and ORFs: a primer on how to analyze derived amino acid sequences. Mill Valley California: University Science Books.
or
McLachlan, A. D. (1971). Test for comparing related amino acid sequences. Mol. Biol. 61, 409-424.

Sequence homology may, but must not reflect *e.g*. that two or more sequences stem from a common origin or are otherwise related.

As outlined above the probability of the interaction of the biomolecules is determined on the basis of the correlation values. Such correlation values may be determined between elements, i.e. sequences, and more particularly distinct positions within these sequences, of the first group and the second group or, alternatively, the first set of data and the second set of data, respectively. These correlation values are referred to herein as group correlation values. These are to be understood as follows: A correlation value reflects similar patterns of variation of two or more positions within one sequence (referred to herein as site correlation values) or between the positions of sequences of two or more biomolecules (referred to herein as group correlation values). Similar patterns of variation are thought to be derived from simultaneous or concurrent events of sequence change along evolution. Such events are believed to reflect compensatory mutations. Herein, the use of the term "correlation value" does not imply the use of any particular algorithm or means of finding or determining such a value. Numerous means of determining correlation values may be applied.

The inventive process and method, respectively, make use of the correlation values, more particularly group correlation values. In a preferred embodiment of the process and method according to the invention this determination may be done as follows: the correlation values are normalized between 0 and 1 and divided in 10 levels. In principle however more or less than 10 levels may be applied.

Preferably, the percentage of correlated pairs, i.e. correlated pairs of sequences of the first and second group of sequences and sequences, i.e. elements, of the first and second set of data, of any correlation values, preferably the correlation values calculated for each alignment are calculated. Even more preferably, the sequences or elements of each group of set of data are aligned and the percentage of correlated pairs is calculated for the alignments of the individual sequences which are thus in some embodiment of the process/method multiple alignments and for the pairs between the two or more alignments. The correlation values are grouped in levels of correlation to calculate the percentage of pairs correlated at each one of the levels. These percentages are used in a preferred embodiment subsequently in that part of the process/method where group correlation values are compared with the correlation values, also the site correlation values, preferably the correlation values of each one of the two alignments.

At each level of correlation the percentage of observations for the combined alignment (which corresponds to the fused sequence, see below) is divided by the sum of the values for the two individual alignments.

The result is multiplied by the value of correlation of the corresponding level. The final value for the prediction of probability of interaction between two biomolecules is obtained as the sum of the values calculated for the individual correlation levels. The probabilities are normalized by the average and standard deviation values (that is Z-score calculation). Average and standard deviations are calculated for the interaction of one biomolecule with all the other possible partners.

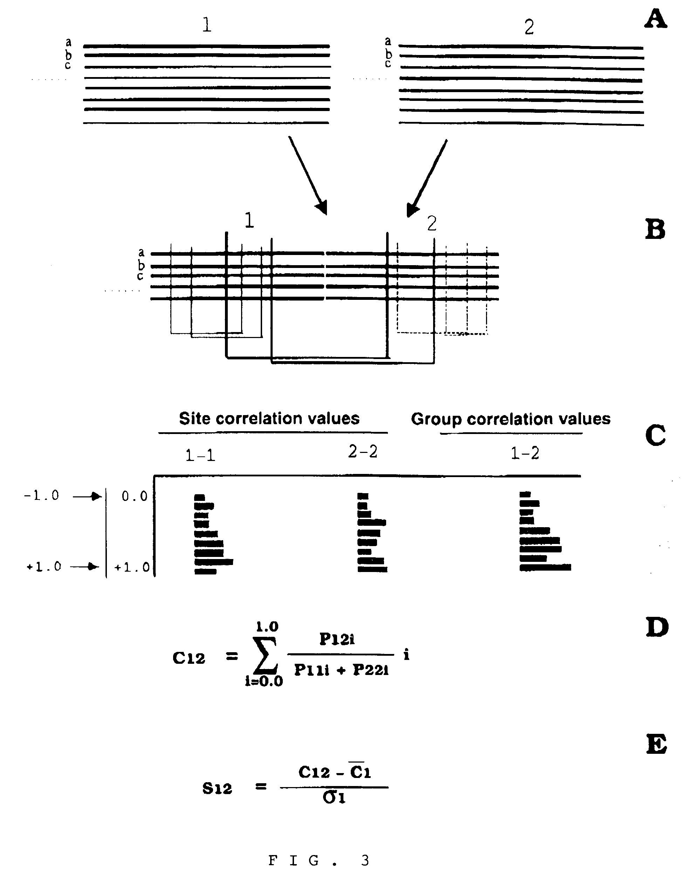

The process outlined above is shown graphically in Fig. 3.

In connection with the present invention the probability that two biomolecules interact is, in a particularly preferred embodiment, calculated based on a predicted interaction value. The calculation of this predicted interaction value is also described in connection with Fig. 3.

A positive predicted interaction value for two (or more) biomolecules means that there is a certain probability that said biomolecules will interact. The higher the predicted interaction value is, the higher the probability that said biomolecules will interact. In other words, the predicted interaction value is a measure for the probability that said biomolecules interact.

There are a multitude of ways of determining correlation values. These ways are known to the one skilled in the art and will be discussed later and are incorporated herein by reference. The process and method, respectively, according to the invention are not limited to one of these and may in fact make use of various different ways and methods, respectively, for determining the probability of the interaction of biomolecules based on correlation values.

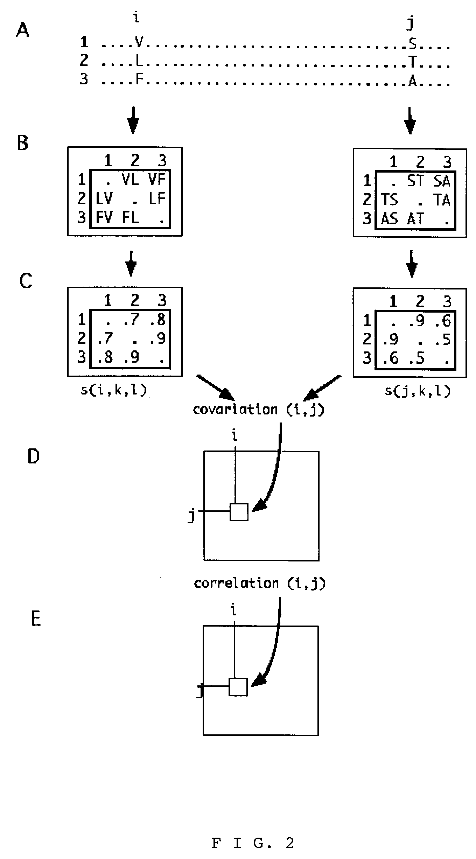

Correlation values may be calculated as outlined in Göbel et al. (Göbel U, Sander C, Schneider R, Valencia A (1994). Correlated mutations and contact in proteins. Proteins 18, 309-317.) and modified to introduce a range correlation calculation. A position specific matrix is calculated for each position in the sequence. This position-specific matrix contains the distances between all sequence pairs at that position. Distances are defined by the scoring matrix of (McLachlan AD , 1971 J. Mol. Biol. 61, 409-424). Positions specific matrices are compared with a covariation coefficient formula that is applied to each of the corresponding values of the position specific matrices. The correlation between each pair of positions is calculated as the average of the covariation values. Fig. 2 outlines graphically the procedure described above.

In Altschuh et al. (Altschuh, D., Lesk, A.M., Bloomer, A.C., Klug, A. Correlation of coordinated amino acid substitutions with function in viruses related to tobacco mosaic virus. J.Mol.Biol.193: 693-707, 1987 and Altschuh, D., Verner, T., Berti, P., Moras, D., Nagai, K. Correlated amino acid changes in homologous protein families. Prot. Engin. 2: 193-199, 1988) correlation values are calculated as simple linear variation of identity patterns between subfamilies.

In Casari et al (Casari, G., Sander, C., Valencia, A., A method to predict functional residues in proteins. Nature Structural Biology. 2 (1995) 171-178) a principal component analysis method is applied to multiple sequence alignments to determine the correlation values between groups of positions.

In Lichtarge et al. (Lichtarge, O., Bourne, H. R., & Cohen, F. E. (1996), An Evolutionary Trace Method Defines Binding Surfaces Common to Protein Families. J. Mol. Biol. 257, 342-358) correlated positions are determined by careful manual analysis of phylogenetic trees in the search of positions clearly related with the main differences between tree branches.

Shindyalov et al. (Shindyalov, I. N., Kolchanov, N. A., & Sander, C. (1994). Can Three-Dimensional Contacts in Protein Structures be Predicted by Analysis of Correlated Mutations. Protein Eng. 7, 349-358) study the variation that accumulate simultaneously in different branches of phylogenetic trees. This method may also be applied for determining correlation values.

Taylor and Harrick (Taylor, W. R., & Harrick, K. (1994), Compensating Changes in Protein Multiple Sequence Alignments, Prot. Eng. 7, 342-348) describe a vector based method for the prediction of correlated mutations in multiple sequence alignments. The method takes into account physical properties and it is more related to the detection of simulatenous variation between different sub-families of proteins.

It is to be noted that any of the correlation values mentioned herein can be calculated in such a manner. Said techniques for calculating correlation values apply thus also to what is called group correlation values as well as to what is called site correlation values.

The methods known in the art for calculating correlation values take as a starting point the individual sequence (of a biomolecule). This applies also in connection with the site correlation values. In other words, the site correlation values are those which are calculated for various positions of a single sequence. This may be performed for any of the sequences in any of the groups or sets of data. The ratio behind this is to reduce the background for the calculation of the group correlation values (see also E.) in Fig. 3).

In a particular preferred embodiment of both the inventive process and the inventive method group correlation values are determined by actually forming one single sequence, which is called a fused sequence, of at least one sequence (or element) of the first group or of the first set of data and at least one sequence (or element) of the second group or of the second set of data. The created fused sequence is then used for the determination of the correlation values. By correlating a position of the fused sequence which stems originally from the first group or set of data, with a position of the fused sequence which stems originally from the second group or of set data, factually group correlation values can be determined.

Also because of this particular approach the determination of the site correlation value which is typically performed before the determination of the group correlation values and used to reduce the background of "wrong" or insignificant group correlation values, allows for the high accuracy of the inventive process and method.

In a preferred embodiment of the present invention the sequences in each group may be present in multiple sequence alignments.

Multiple sequence alignments used herein, refer to the alignment of DNA, RNA or amino acid sequences based on their sequence similarity. Such an alignment may be done manually or with the aid of a computer making use of an algorithm or method *e.g.* such algorithms or methods are *e.g.* the BLAST algorithm (Altschul, S.F., Gish, W. Miller, W., Myers, E.W., and Lipman, D.J., J. Mol. Biol. 215, 403-410 (1990) or an algorithm by Altschul (Altschul, S.F. (1993) "A protein alignment scoring system sensitive at all evolutionary distances." J. Mol. Evol. 36:290-300), the CLUSTALW method (Higgins, D. G., Bleasby, A. J., & Fuchs, R. (1992)), CLUSTAL V (Improved software for multiple sequence alignment. Comput. Appl. Biosci. 8, 189-191), or the MAXHOM method (Sander, C. & Schneider, R. (1991), Database of homology-derived structures and the structural meaning of sequence alignment, Proteins 9:56-68) but are not limited to these.

In a preferred embodiment of the present invention such alignments are generated using the Altschul algorithm (Altschul, S. F. (1993). A Protein Alignment Scoring System Sensitive at All Evolutionary Distances. J. Mol. Evol. 36, 290-300).

It should be noted that the above mentioned means of aligning sequences are examples or preferred embodiments. The process according to the invention can be realized using any method of sequence alignment.

In a preferred embodiment of the present invention additionally site correlation values for substitutions within the sequences within the first group or first set of data are determined, and additionally site correlation values for substitutions within the sequences within the further second group(s) or set(s) of data are determined. According to the invention such site correlation values may be used to determine the statistical significance of the group correlation values determined.

In a preferred embodiment of the process according to the invention the site correlation values within the groups are determined prior to the determination of the group correlation values.

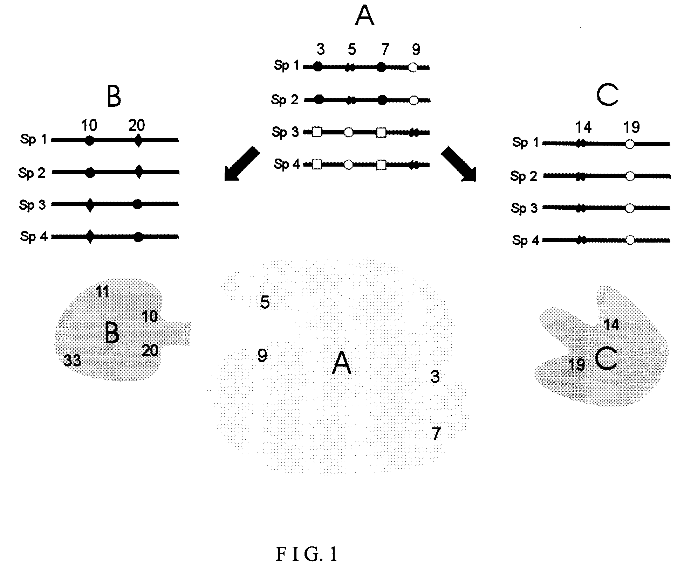

A graphical representation of a possible embodiment of the process according to the present invention is depicted in Fig. 1.

The process and method according to the present invention also make use of one or more sequences. Such sequences may be RNA, DNA or amino acid sequences. They may be known, unknown i.e. generated de novo, publicly available or not. The sequences may be of natural origin or artificially generated, they may but must not represent genes or parts of genes. The sequences may have any given length.

Given one or more alignments to be used for the process according to the present invention it is desirable if each position in the alignment is coded by a distance matrix. It may be preferred if the distance matrix used is from McLachlan.

This position specific matrix contains all the residue-residue distances between all possible pairs of sequences at that position. These possible pairs may be pairs with both components of the pairs originating from a single group or set of data or with one component of the pairs originating from the first group or first set of data and the other component originating from the or any second group or set of data. In the event that a single fused sequence is created, pairs are also created. In this case the last residue of the fused sequence number 1 and the first residue of the fusion partner sequence number 2 are known thereby allowing for the above mentioned distance determination of the pairs of positions. Distances between amino acids may be defined by the scoring matrix of McLachlan (McLachlan, A. D. (1971). Test for comparing related amino acid sequences. J. Mol. Biol. 61, 409-424) or another scoring matrix.

The correlation value between each pair of positions is calculated as the average of the correlation for each corresponding bin of the position specific matrices. Corresponding bins contain the distance between the same two sequences in the two positions under comparison.

Bin as used herein means equivalent position in a matrix which is determined by a row and a column. The calculation implies comparing the corresponding positions of two "position specific matrices" calculating the covariation value for them and then averaging all the covariation values corresponding to each one of the different positions in the matrices (bins).

The DNA, RNA or amino acid sequences may stem from known or unknown organisms, may be created artificially, may represent sequences that are in parts from living or dead organisms and in other parts artificially created. The sequences may be newly determined using biochemical methods or may be taken from existing databases.

In a preferred embodiment the sequences represent genes of an organism. In another preferred embodiment the sequences represent the translated genes of an organism.

Most preferably the sequences are amino acid sequences and represent genes from an organism.

The process and method according to the present invention also make use of groups of sequences. In one embodiment of the invention such groups comprise at least 2 sequences. In an even more preferred embodiment of the invention such groups comprise at least 11 sequences. It should be noted that the more sequences are used the better the results achieved may become.

In a preferred embodiment of the present invention sequences in the groups stem from a multitude of different origins such as species, tissues or organisms representing a majority of sequence space and/or very distantly related species. Preferably one may want to align a number of sequences that are very similar that is about 50% similar or more, as well as sequences that are much less similar.

Herein, similar sequences are such sequences which are alignable when applying the CLUSTALW method (Higgins et al, see above) and such sequences that fall under the description of related sequences or derived sequences as found in
Doolittle, R. F. (1986). Of URFs and ORFs: a primer on how to analyze derived amino acid sequences. Mill Valley California: University Science Books.
or
McLachlan, A. D. (1971). Test for comparing related amino acid sequences. Mol. Biol. 61, 409-424.

Groups comprising very similar sequences as well as not very similar sequences, herein very similar are those sequences in which over 50 % of the residues are identical and not very similar sequences are sequences in which 20 % or less of the residues are identical, are a preferred group for performing the process according to the invention.

In a preferred embodiment it is particularly advantageous if the sequences represented in the first group or set of data, the group containing homologous of the sequence representing biomolecules for which an interacting biomolecule is to be determined, stem from the same origin *i.e.* species, tissues or organisms as those sequences or elements in the second group or second set of data and *vice versa.* If, for example, origin means species the homologous biomolecules or their sequences may stem from different kinds of tissues of a single species such as liver or heart. If, as a further example, origin is to mean tissue, the homologous biomolecules or their sequences may stem from the same tissue of different species.

In a further preferred embodiment of the present invention it is desirable if the homologous sequences represent homologous genes. These genes may be represented by their amino acid sequence, their DNA sequence or their RNA sequence. A gene herein is to be understood as a DNA sequence that is transcribed into RNA *in vivo,* or a DNA or RNA sequence that encodes a polypeptide *in vivo.*

One can distinguish between orthologues sequences and paralogues sequences. Herein orthologues sequences are those which show close similarity between species and share a common evolutionary origin and paralogues sequences are those sequences which show close similarity within species, indicative of a close evolutionary relationship which may or may not have pre-dated speciation. The present invention may make use of both of these types of sequences. In a preferred embodiment of the present invention however, the process according to the invention makes use only of orthologues sequences.

The present invention can be performed manually or by using a computer. In a preferred embodiment of the present invention the data i.e. the sequences and/or the groups a) and/or b) of sequences are present in a computer readable form.

It is to be noted that what has been said in connection with the inventive process in principle also applies to the inventive method.

The inventive method may be used for the simulation of biomolecule interaction. This simulation is actually based on knowing which biomolecules may interact which each other. Insofar, the inventive method provides this prerequisite for the simulation. The simulation itself may then deduce from the sequence of the interacting biomolecules suitable representations such as three dimensional models to visualize the interaction.

The inventive device for simulating the interaction of biomolecules comprises, among others, a loading device and a processing device as well as an output device. The loading device may retrieve the sets of data required to perform the inventive method, e.g., from any kind of data-bank, analytical instrument, individual files, or textual information. This includes also retrieval of respective sets of data, i.e. sequences, from the internet. The processing device is then responsible for performing the inventive method and comprises preferably a computer. The processing device provides for output data which in turn are received from an output device for further handling of said output data. The output data may then be stored on any suitable medium, be printed out, written to a further document or be submitted to further processing.

Said device may also transfer the output data generated by the processing device making use of the inventive method as output control characters to, e.g. a further computer to perform the next step of the simulation of the interaction of the particular biomolecules where the fact that said biomolecules interact with each other, is carried out.

The present invention will further be illustrated by examples wherein
Fig. 1 illustrates the process by which a biomolecule A is analyzed with respect to interacting with biomolecules B and C;
Fig. 2 is a graphical representation of an embodiment of the proposed process for calculating correlated mutations;
Fig. 3 is a schematic representation of possible parts of the process according to the present invention;
Fig. 4 shows the results of the application of the process according to the invention; and
Fig. 5 shows the result of a determined interaction of biomolecules making use of the i-2-hybrid process.

Fig. 1 illustrates the process by which a biomolecule A is analyzed with respect to a possible interaction with biomolecules B and C in order to determine the most likely interaction partner. The process according to the present invention determines that the pattern of variation of two positions of the sequence biomolecule A (position 5 and 9) are similar and that they are at the same time similar to the patterns of variation of positions 10 and 20 of the sequence of protein B. No other positions are similar to them, for example none of the positions of the sequence of biomolecule C have patterns of variation similar to positions in protein A. Therefore the process according to the invention determines a proposed interaction between biomolecules A and B based on the possible interactions of positions 5 and 9 of sequence A with positions 10 and 20 of sequence B.

Fig. 2 is a graphical representation of an embodiment of the proposed process for calculating correlated mutations (Göbel et al., 1994). In A) a protein family is presented as a multiple sequence alignment (series of horizontal lines, where the numbers 1 to 3 represent different sequences (indices k, 1 run over proteins in the family) and the indices i and j run over positions in the alignment. Mutational behavior at each single position is summarized in a matrix B), including all the possible comparisons of the different sequences at that position. The position specific matrix C) is derived from B) according to a standard table of distances, *e.g.* McLachan (1971). In D) the covariation value is calculated for each one of the corresponding sequence pairs (k, 1). Finally in E) the correlation value is calculated as the average of the covariation values of the two positions (i, j) and it carries information about the level of similarity of the mutational patterns of the two positions.

Fig. 3 shows a schematic representation of possible parts of the process according to the present invention. In A) sequences from different species (a, b, c, ....) are collected for two different biomolecules 1 and 2. The sequences are expected to correspond to the same species. In B) a virtual alignment is constructed concatenating the sequences of each one of the species for biomolecule 1 and 2. This concatenation leads to fusion sequences. The site correlation and group correlation values are calculated according to the procedure described above. In C) the correlation values are scaled (into correlation slots) between 0 and 1 and the frequency of the different pairs of residues in the different correlation slots recorded. In the example the correlation values have been distributed in nine different correlation slots and the corresponding frequency distribution are represented for the two biomolecules (P11 and P22) and for the group correlation values (P12).

D.) The distribution of site correlation values and the distribution of group correlation values are compared using the given formula. The correlated mutational behavior between the two biomolecules (C12) is calculated as the sum for the different correlation slots (i) of the ratio between distribution of values for the group correlation values (P12i) and the distribution of values for the site correlation values for the two biomolecules (P11i and P22i). This ratio is weighted with the value of the corresponding correlation slot (i) in a way that high values of correlation are given more importance.

E.) The predicted interaction value or predicted interaction score for the interaction between biomolecules 1 and 2 is calculated as a Z-score S12 of the C12 value relative to all interaction values for biomolecule 1. In the formula represented by C12 minus the mean of all interaction values for biomolecule 1 over the standard deviation of the group correlation values of the biomolecule 1 with all the other possible partners in the test set. The predicted interaction value is given in terms of standard deviation, positive values indicate a positive predicted interaction and the strength of the value the likelihood of the interaction.

Generally, the establishment of slots, more particularly correlation slots (i) refers to a process in which we take on the one hand the pairs of positions with the correlation value for the two possible alignments (site correlations), group them according to their value of correlation, count how many we have in each of the groups (for example, how many pairs in protein 1 are between 0.1 and 0.2 or correlation value) on the other hand we do the same for the pairs of position which form the alignment and the other forming the other (that is the pairs of the (group correlation). In this case we would be counting the number of pairs with one residue in protein 1 and another in protein 2, with a value of correlation between lets say 0.2 and 0.3. The process then is to compare the precentages of pairs at the different levels of correlation (the 0.2 to 0.3 level above) for the individual alignments site correlations with the percentage of pairs at the same level for the group correlation values. This is what is given in the formula.

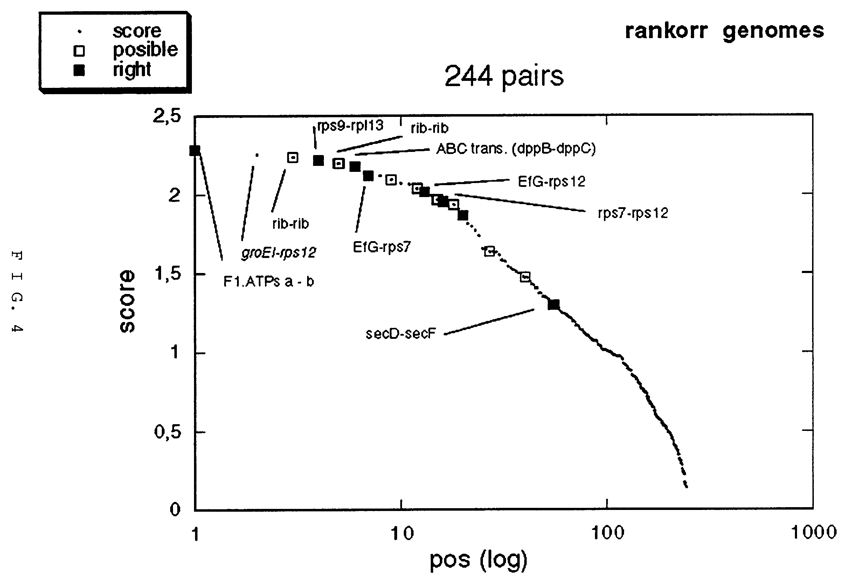

Fig. 4 shows the result of the application of the process according to the invention (as exemplified in example 1) using sequences obtained from full genomes. The interaction probabilities are given in the Y-axis and proteins pairs are sorted according to these values (X-axis, logarithmic scale). The names of some of the proteins are indicated. The pairs of proteins known to interact are represented by filled symbols, those possible interactions corresponding in many cases to proteins that form part of complexes are given with open squares, most likely non-interacting proteins are represented by dots.

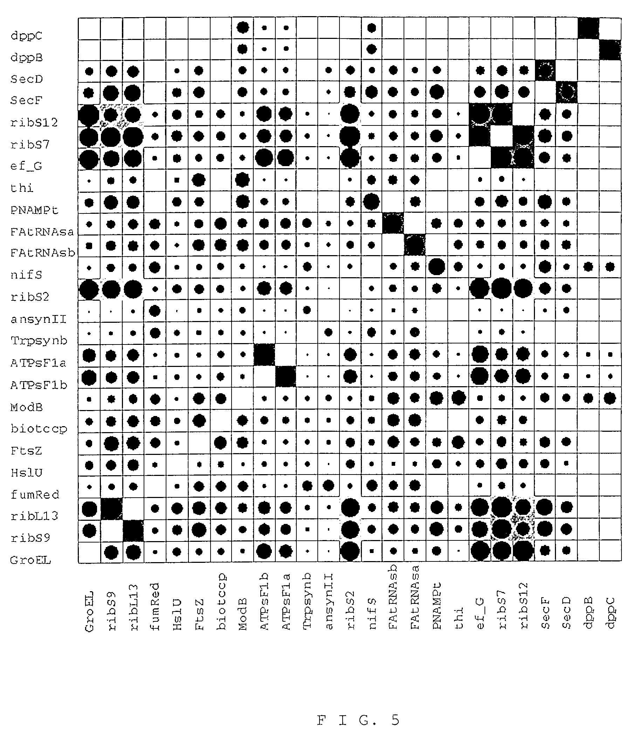

Fig. 5 shows the result of determined interaction of biomolecules making use of the i-2-hybrid (as specified in example 1) process depicted in a way reminiscent of the "wet" experimental yeast two hybrid system. Here, the diameter of the dots is proportional to the probability of interaction as determined using the process of the invention. In this case the minimal level of correlation entered in the analysis was of 0.4. The names of all the proteins used in the analysis are indicated. The empty squares correspond to those cases in which it was impossible to identify sequences from at least 11 species in common for those two proteins. The well known interacting proteins are highlighted with a dark square and the possibly interacting ones with a light-shaded square (*e.g.* different ribosomal proteins and elongation factors).

### Examples:

### Example 1 (Fig. 4 and 5):

The process according to the present invention was demonstrated by picking the right pair of interacting proteins in different sets of multiple sequence alignments. The multiple sequence alignments were generated using the ClustalW algorithm (Higgins, D. G., Bleasby, A. J., & Fuchs, R. (1992)).

A set composed of 53 proteins was analyzed. The sequences homologous to each one of them were collected from 14 different microbial genomes, that are completely sequenced and publicly available. The group correlation values were calculated for 244 pairs of proteins, that had at least 11 sequences from the same species in common.

In this set seven of the pairs of proteins with well documented interactions were among the ones with high predicted probability of interaction based on the group correlation values. And additional set of ten pairs of proteins with high probability of interaction correspond to possibly interacting proteins as, for example, different ribosomal proteins. This high probability can be taken from or is expressed as a positive predicted interaction value herein.

Only one pair of proposed interacting biomolecules (SecD and SecF) has a relatively low value but has previously been described to represent interacting proteins. Interestingly, this probability is still better than any of the other probabilities calculated for these two proteins with the other alternative interaction partners in the set. The results of these experiments may be seen in figures 4 and 5.

### Example 2:

Utilising the i-2-hybrid process we have constructed a database containing all the predicted interactions for *E. coli* proteins for which enough alignments were found in 20 complete bacterial genomes (4289 proteins as basic entries in the database). For this set of proteins it was possible to compute the interaction for 67238 pairs for which enough sequences of common species were detected. Each one of the entries is indexed and linked to other databases, in particular to Swissprot. The data base contains all the possible partners in each interaction and the reliability value of this interactions.

The quality of the predictions of interacting proteins in the database will benefit from continuous updates and from the continuous increase in the number of known sequences, in two ways. In the first place, the number of sequences that can be included in the alignments will raise the possibility of identifying interacting biomolecules and in the second place it will increase the reliability of the predictions since the basic methods often work better using alignments with many sequences.

Among the high scoring protein pairs determined by the i-2-hybrid process a number have previously been shown experimentally to interact as well, including membrane transporters of related compounds (G1787080-G1787369), transcriptions factors implicated in the control of related functions (G1787229-G1790863) or different subunits of an enzyme (G1787748-G2367325). As with other methods also false results may occur as the pair at position 10 (G1786981-G1790408) a transporter predicted to interact with a transcription factor/enzyme.

The database contains many interesting predictions of interaction, that in some cases could provide for a first clue with respect to the function of some proteins. For example among those comparisons resulting in positive predicted interaction values it is possible to find some proteins of known function like two transmembrane proteins (G1786670-G2367355) belonging to the (UPF0005) family, or another pair formed by a transcription factor implicated in the nitrate/nitrite response regulation that is predicted to interact with a protein of unknown function, that by homology looks similar to other transcription also implicated in nitrite/nitrate response regulation.

The database is organized to detect interactions for one given organism, in this case it is specific for *E. coli,* since the interactions are predicted in basis to the family alignments for a given organism.

The data contained in the newly generated database can for example be queried by protein names, gene names or accession numbers.

The features disclosed in the foregoing description, in the claims and/or in the accompanying drawings may, both separately and in any combination thereof, be material for realising the invention in diverse forms thereof.

## Claims

1. Process for the determination of interacting biomolecules characterized in that similar patterns of variation between two or more positions of at least two biomolecules are used.

2. Process for the determination of interacting biomolecules,
characterized in that
a) a first group is provided comprising sequences representing homologous biomolecules,
b) at least one second group is provided comprising sequences representing homologous biomolecules,
c) group correlation values between the sequences of the first group and the sequences of at least one second group are determined, and
d) the probability of the interaction of the sequence represented biomolecules is determined on the basis of the group correlation values.

3. Process according to claim 2, characterized in that the probability of the interaction is calculated as predicted interaction value.

4. Process according to claim 2 or 3, characterized in that the interacting biomolecules are those with a positive predicted interaction value.

5. Process according to any of claims 2 to 4, characterized in that any of the second group(s) is converted into the first group and the first group is converted into a second group and group correlation values between the sequences of this new first group and the sequences of any of the second group(s) which also comprises the former first group, are determined.

6. Process according to any of claims 2 to 5, characterized in that site correlation values within each of the sequences within the first group and/or site correlation values within each of the sequences within the second group(s) are determined and said site correlation values are used for the calculation of the probability of interaction and/or for the calculation of the predicted interaction value of the sequence represented biomolecules.

7. Process according to claim 6, characterized in that the site correlation values are correlation values for substitutions within the sequences

8. Process according to any of claims 2 to 7, characterized in that
each sequence of each of said groups is fused to each other to form fused sequences comprising at least one sequence of the first group and at least one sequence of any second group(s),
the correlation values within these fused sequences are determined, and
the correlation values are used as group correlation values for determining the predicted interaction value and/or the probability of interaction.

9. Process according to any of claims 2 to 8 characterized in that
correlation values are determined by
creating a position specific matrix containing the distances between pairs of sequences at that position whereby the distances are calculated by applying a standard distances matrix,
creating a combined matrix for two positions by calculating the covariation coefficient between equivalent positions of their position specific matrices, and
determining the correlation value for a pair of positions by averaging the correlation values of the combined matrix.

10. Process according to claim 9, characterized in that the standard distances matrix is the scoring matrix by McLachlan.

11. Method for the determination of interacting biomolecules which comprises processing data of at least a first set of data and at least a second set of data to output data
wherein each of the sets of data comprises independently and individually at least one or more elements,
wherein each of the elements represents the sequence of a biomolecule,
wherein the elements of a single set of data represent a group of homologous biomolecules,
wherein the output data comprises at least one pair of elements with one part of the pair of elements comprising at least one element from the first set of data and the other part of the pair of elements comprising at least one element from the second set of data,
characterised in that
- a group correlation values data set is created comprising group correlation values which are determined between the sequences of the first set of data and at least the second set of data;
- an interaction probability data set is created by retrieving group correlation values from the group correlation values data set and determining the probability of interaction of the biomolecules based on the group correlation values; and
at least some of the elements from the first and at least the second set of data which have been used to create the group correlation values and the interaction probability therefrom form the output data.

12. Method according to claim 11, characterized in that the probability of the interaction is calculated as predicted interaction value.

13. Method according to claim 11 or 12, characterized in that the elements the predicted interaction value of which is positive, are interacting biomolecules.

14. Method according to any of claims 11 to 13, characterized in that
any of second set(s) of data is converted into the first set of data and the first set of data is converted into a second set of data, and
group correlation values are determined between the sequences of this new first set of data and the sequences of any of the second set(s).

15. Method according to any of claims 11 to 14, characterized in that
site correlation values within each of the sequences within the first set of data and/or site correlation values within each of the sequences within the second set(s) of data are determined, and
said site correlation values form a set-specific site correlation value data set.

16. Method according to claim 15, characterized in that the set-specific site correlation value data set is used to calculate the probability of interaction of and/or to calculate the predicted interaction value of the sequence represented biomolecules.

17. Method according to claim 15 or 16, characterized in that the site correlation values are correlation values for substitutions within the sequences.

18. Method according to any of claims 11 to 17, characterized in that
a fused element set of data is generated by combining each element of the first set of data individually with each element of any of the second set(s) of data, and
attributing each fused element individually to the fused element set of data.

19. Method according to claim 18, characterized in that
the correlation values are determined within the various positions of a single element of the fused element set of data, and
the correlation values are used as group correlation values for determining the probability of the interaction of and/or predicted interaction value(s) of the biomolecules.

20. Method according to any one of claims 11 to 19, characterized in that the correlation values are determined by
creating a position specific matrix containing the distances between pairs of sequences at that position whereby the distances are calculated by applying a standard distances matrix,
creating a combined matrix for two positions by calculating the covariation coefficient between equivalent positions of their position specific matrices, and
determining the correlation value for a pair of positions by averaging the correlation values of the combined matrix.

21. Method according to claim 20, characterized in that the standard distances matrix is the scoring matrix by McLachlan.

22. Method according to any of claims 11 to 21, characterized in that the first set of data and/or second the second set(s) of data are retrieved from a medium which is selected from the group comprising databanks, linked databanks, textual data and sets of data generated by an analytical instrument.

23. Method according to any of claims 11 to 22, characterized in that the set(s) of data comprise alligned sequences.

24. Method according to any of claims 11 to 23, characterized in that the output data are output control characters for a target medium.

25. Method or process according to any of claims 2 to 24, characterized in that the sequences of the first group or second group(s) or first set of data or second set(s) of data are selected from the group comprising DNA sequences, RNA sequences and amino acid sequences.

26. Method or process according to any of claims 2 to 25, characterized in that the number of sequences comprised in any of the groups or any of the sets of data is at least , preferably at least 11.

27. Method or process according to any of claims 2 to 26, characterized in that the sequences are homologous sequences.

28. Method or process according to claim 27, characterized in that the homologous sequences stem from different origins.

29. Method or process according to claim 27, characterized in that the homologous sequences in the first set of data and in the second set of data stem from the same origin and/or the homologous sequence in the first group and in the second group stem from the same origin.

30. Method or process according to any of claims 27 to 29, characterized in that the homologous sequences are homologous genes.

31. Method or process according to claim 30, characterized in that the homologous genes are orthologs.

32. Use of the method according to any of claims 11 to 31 for the simulation of biomolecule interaction.

33. Use according to claim 32 wherein the interacting biomolecules are those with a positive predicted interaction value determined by a process or method according to any of the preceeding claims.

34. Pairs of interacting biomolecules determined according to a method or process according to any of the claims 2 to 31.

35. Data structure readable by a computer, said data structure being generated by a process or a method according to any of claims 2 to 31.

36. Computer readable medium for embodying or storing therein data readable by a computer, said medium comprising one or more of the following:
a data structure generated by executing a process or a method according to any of claims 2 to 31;
Computer program code means which is adapted to cause a computer to execute a process or method according to any one of claims 2 to 31.

37. Computer program product comprising the computer readable medium according to claim 36.

38. Database containing information on interacting sequence pairs generated by applying the process or method according to any of the claims 2 to 31.

39. Database according to claim 38, wherein the database is an organism/species specific database.

40. Computer system comprising an execution environment for running the process or method according to any of the claims 2 to 31.

41. Device for simulating the interaction of biomolecules represented by their sequences which comprises
a loading device for making available the sets of data according to any of the claims 11 to 31,
a processing device for performing the method according to any of the claims 11 to 31,
an output device for receiving the output data generated by the processing device.
